(19) Europäisches Patentamt / European Patent Office / Office européen des brevets

(11) EP 2 146 683 B1

# (12) FASCICULE DE BREVET EUROPEEN

(45) Date de publication et mention de la délivrance du brevet:
**12.08.2015 Bulletin 2015/33**

(21) Numéro de dépôt: **08736559.9**

(22) Date de dépôt: **24.04.2008**

(51) Int Cl.:
*A61K 8/21* (2006.01) *A61K 8/69* (2006.01)
*A61K 8/97* (2006.01)

(86) Numéro de dépôt international:
**PCT/EP2008/055032**

(87) Numéro de publication internationale:
**WO 2008/132149 (06.11.2008 Gazette 2008/45)**

## (54) UTILISATION D'UN EXTRAIT AQUEUX DE PEPINS DE RAISIN EN ASSOCIATION AVEC AU MOINS UN SEL DE FLUOR CONTRE LA FORMATION OU L'ACCUMULATION DU BIOFILM DENTAIRE ET LES COMPOSITIONS COMPRENANT CETTE ASSOCIATION

VERWENDUNG EINES WÄSSRIGEN TRAUBENKERNEXTRAKTS IN KOMBINATION MIT MINDESTENS EINEM FLUORSALZ ZUR BEKÄMPFUNG DER BILDUNG ODER AKKUMULATION EINES ZAHNBIOFILMS UND DIESE KOMBINATION ENTHALTENDE ZUSAMMENSETZUNGEN

USE OF AN AQUEOUS GRAPE SEED EXTRACT COMBINED WITH AT LEAST ONE FLUORINE SALT TO COMBAT THE FORMATION OR ACCUMULATION OF DENTAL BIOFILM AND COMPOSITIONS COMPRISING SAID COMBINATION

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **24.04.2007 FR 0754670**

(43) Date de publication de la demande:
**27.01.2010 Bulletin 2010/04**

(73) Titulaire: **Pierre Fabre Médicament**
**92100 Boulogne-Billancourt (FR)**

(72) Inventeur: **VEZIN, Jean-Claude**
**F-81580 Soual (FR)**

(74) Mandataire: **Regimbeau**
**20, rue de Chazelles**
**75847 Paris Cedex 17 (FR)**

(56) Documents cités:
**EP-A- 1 072 254**
**DE-A1- 19 949 575**
**FR-A- 2 318 632**
**US-A- 5 470 565**
**US-A1- 2003 103 914**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 146 683 B1

## Description

La présente invention concerne l'association d'un extrait aqueux de pépins de raisin en association avec un sel de fluor pour son utilisation en tant que composition anti-adhésion d'hygiène buccale destinée à prévenir et/ou diminuer la formation et l'accumulation du biofilm dentaire sur les surfaces dentaires ou le tissu épithélial gingival, ainsi que les maladies bucco-dentaires associées, tout en préservant l'équilibre de l'écosystème buccal.

La cavité buccale constitue une véritable niche écologique par laquelle transitent et séjournent la salive, les aliments, l'air et les produits bactériens.

Dans la cavité buccale, les bactéries peuvent adopter deux modes de vie radicalement différents : soit celui à l'état planctonique, dans lequel les organismes isolés flottent dans le milieu buccal, soit celui où dans un biofilm, les bactéries attachées à une surface dentaire vivent en communauté.

La santé dentaire et parodontale peut être considérée comme un état d'équilibre dans lequel la population bactérienne coexiste avec l'hôte et où aucun dommage irréparable n'apparaît dans les tissus de ce dernier. Toutefois, la maladie peut apparaître quand la composition et les activités métaboliques des communautés du biofilm dentaire sont perturbées.

Les lésions du parodonte apparaissent d'abord au niveau de la gencive, provoquant des gingivites, inflammations de la gencive marginale. Puis, elles peuvent évoluer en parodontites affectant l'ensemble du parodonte et deviennent irréversibles en l'absence de traitement.

Il existe différentes formes de gingivites :

- gingivite associée à la présence de plaque dite banale de négligence,
- gingivite aiguë ulcéro-nécrotique,
- gingivite dont l'origine n'est pas liée à la plaque :

    o associée à des maladies cutanée ;
    o limitée à la gencive ou intéressant l'ensemble de la muqueuse buccale ;
    o allergique ;
    o infectieuse.

Le biofilm dentaire (ou plaque dentaire) est une accumulation hétérogène, adhérente à la surface des dents ou située dans l'espace gingivo-dentaire, composée d'une communauté microbienne riche en bactéries aérobies ou anaérobies, enrobée d'une matrice intercellulaire de polymères d'origine microbienne et salivaire. Il s'agit d'une organisation bactérienne complexe dont les premiers stades de formation correspondent à un dépôt de glycoprotéines sur les surfaces des tissus durs ou des tissus mous baignant dans la salive. Cette première couche porte le nom de pellicule exogène acquise (PEA) ou encore « biofilm salivaire ». Elle est secondairement colonisée par des micro-organismes qui vont s'organiser en fonction de critères physicochimiques, nutritionnels ou relationnels et constituer ainsi un dépôt mou, adhérent, tenace, et de couleur blanc jaunâtre, à la surface des dents et des matériaux dentaires couramment utilisés.

On distingue deux types de plaque définis en fonction de leur localisation anatomique par rapport à la gencive : la plaque supragingivale et la plaque sous-gingivale. Ces deux types de plaques constituent deux micro-environnements différents. L'environnement supragingival est baigné par la salive, tandis que l'environnement sous-gingival est baigné par le fluide du sillon gingival. L'environnement supragingival est surtout aérobie, alors que l'environnement sous-gingival est presque exclusivement anaérobie. L'espace sous-gingival a la forme d'un cul-de-sac, sans chasse liquidienne, et les forces mécaniques susceptibles de désagréger les populations bactériennes établies y sont rares. Au contraire, les zones supragingivales sont balayées continuellement par la salive, exposées à tous les mécanismes d'attrition propres à la cavité buccale (mastication, déglutition, phonation) et directement accessibles aux mesures d'hygiène.

La plaque supragingivale et la plaque sous-gingivale se distinguent également par leur potentiel pathogénique : la plaque supragingivale est spécifiquement impliquée dans la pathologie carieuse alors que la plaque sous-gingivale est associée aux pathologies gingivales et parodontales. Les principales bactéries retrouvées dans la plaque supragingivale sont *Streptococcus mutants, Streptococcus sanguis, Streptococcus mitis, Streptococcus sobrinus, Lactobacillus sp., et Actinomyces sp. (A. viscoses).* En fonction de sa situation côté dent ou côté épithélium, la composition de la flore sous-gingivale varie de façon importante. On note la présence de *Porphorymonas gingivalis* et de *Fusobacterium nucleatum*, qui sont ainsi fortement impliquées dans les pathologies parodontales.

Les agents antiseptiques bactéricides, sont souvent utilisés dans des compositions pour réduire la plaque dentaire. Toutefois les agents bactéricides ne sont pas complètement satisfaisants car leur action antiseptique forte peut induire un déséquilibre au sein de la population bactérienne naturelle de la cavité buccale. En effet, les produits antiseptiques agissent sur les microorganismes :

- de manière drastique : l'évaluation de l'activité étant basée sur une notion de réduction logarithmique, soit une destruction ou une inactivation d'un nombre élevé de microorganismes,
- rapidement : en considérant un temps de contact court et une inactivation de la plupart des molécules au contact notamment des matières organiques.

De plus, leur utilisation dans le temps doit être limitée car ils peuvent favoriser la sélection de souches résistantes.

Il existe donc un besoin de développer des agents capables de limiter ou supprimer le dépôt du biofilm par une action anti-adhésion, et permettant ainsi de conserver une bonne santé bucco-dentaire sans effets secondaires et sans perturber l'équilibre de l'écosystème buccal. Ce concept est basé sur une action non pas au niveau de la croissance ou de la viabilité cellulaire mais au niveau des interactions de la cellule bactérienne avec l'ensemble des surfaces buccales : surfaces dures (ex. émail dentaire), muqueuses, ainsi qu'entre cellules bactériennes.

Il a été découvert de manière surprenante que de tels agents anti-biofilm respectueux de l'équilibre de la flore bactérienne buccale peuvent être extraits des pépins de raisin.

Le raisin contient de nombreux principes actifs, dont les polyphénols. Les pépins de raisin renferment surtout des polyphénols de type oligo-proanthocyanidines (OPC) qui sont connus pour leur fort pouvoir antioxydant.

Les extraits de pépins de raisins sont indiqués dans le traitement de l'insuffisance veineuse et des varices, pour atténuer le stress oculaire causé par l'éblouissement, ou pour accélérer la guérison des tuméfactions consécutives à une blessure ou à une chirurgie.

Les OPC de pépins de raisin sont aussi utilisés dans le domaine de la cosmétologie comme agent anti-radicalaire pour lutter contre le vieillissement de la peau.

L'utilisation d'extraits de plantes riches en polyphénols pour leur action anti-bactérienne, en particulier pour l'hygiène buccale, par exemple pour la prévention de la parondontite est décrite dans EP 1 072 254. Cependant, ce document ne décrit pas le problème technique de la présente invention, ni même l'utilisation d'extraits de pépins de raisin.

En outre, le document US 5,470,565 divulgue quant à lui l'utilisation d'une association de polyphénols de thé avec un sel de fluor pour renforcer la résistance acide des dents.

Par ailleurs, le document FR 2 318 632 décrit l'utilisation de sels de fluor seuls dans la prévention et le traitement des parodontopathies.

DE 199 49 575 décrit une combinaison de flavonoïdes avec des fluorures d'ammonium pour lutter contre les bactéries, problème technique différent de celui de la présente invention, consistant à fournir une composition destinée à l'hygiène buccale permettant de lutter contre le biofilm dentaire en apportant une action anti-adhésion et non par une action classique antiseptique forte. Ainsi, l'activité anti-adhésion des compositions décrites dans DE 199 49 575 n'est ni mentionnée ni évaluée. En outre, les flavonoïdes utilisés dans ce document sont des flavonoïdes de synthèse purs, et rien ne suggère qu'un extrait de pépins de raisin serait aussi efficace.

Le document US 2003/0103914 décrit des compositions de soin buccal stables visant à combattre l'halitose comprenant :

- un extrait de pépins ou de pulpe de la famille des Citrus (agrumes) ou Vitis (raisins) et leurs mélanges,
- un agent actif de soin buccal choisi parmi les agents anti-tarte, anti-plaque les ions fluorures, les agents de désensibilisation, les agents contre la mauvaise haleine, les antagonistes H2, et leurs mélanges
- un excipient pharmaceutiquement acceptable.

Cependant, seuls les effets anti-bactériens des extraits des plantes de la famille des Citrus et Vitis sont décrits dans ce document (voir [0011] à [0013]). Ainsi, ce document ne mentionne pas le problème technique de la présente invention.

US 2003/0103914 propose un très large choix (plusieurs dizaines) d'agents à combiner avec les extraits de plantes mentionnés plus haut, et oriente l'homme du métier vers les polyphosphates ([0053], [0056] et revendication 12), les sels métalliques (revendications 13 à 16, [0005], [0063] à [0065]), et non vers les sels de fluor.

Il a été découvert de manière surprenante que les extraits de pépins de raisin peuvent être utilisés dans le domaine de l'hygiène bucco-dentaire comme agents antibiofilm.

Les inventeurs ont par ailleurs découvert de manière surprenante que l'action anti-biofilm de l'extrait de pépins de raisin est potentialisée par les sels de fluor, tels que le fluorure de sodium ou le fluorinol (fluorhydrate de 3-pyridyl méthanol).

La présente invention a pour objet l'association d'un extrait aqueux de pépins de raisin en association avec au moins un sel de fluor pour son utilisation en tant que composition d'hygiène buccale destinée à :

prévenir et/ou diminuer la formation ou l'accumulation du biofilm dentaire sur les surfaces dentaires ou le tissu épithélial gingival
tout en préservant l'équilibre de la flore bactérienne.

Les sels de fluor selon la présente invention sont de préférence des fluorures inorganiques, des fluorures d'amines ou des fluorures d'ammonium quaternaire.

Les fluorures inorganiques peuvent être notamment choisis parmi les fluorures de potassium, de sodium et d'étain, ainsi que leurs mélanges.

Les fluorures d'amine sont de préférence choisis parmi les sels répondant à la formule (I) suivante :

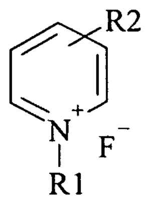

(I)

dans laquelle :

* R1 = H, alcoyle ;

* R2 = $-CH_2-OH$, carboxylate d'alcoyle, $-CO-NH-CH_2-CH_2-OH$,

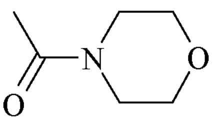

;

et R2 se trouve en position 2 ou 3.

L'extrait aqueux de pépins de raisin selon la présente invention présente la caractéristique d'être riche en polyphénols, notamment en proanthocyanidines (OPC).

La fraction massique de polyphénols dans ledit extrait est de préférence supérieure à 50 %, de préférence supérieure à 90 %.

La fraction massique de proanthocyanidines dans ledit extrait est de préférence comprise entre 20 et 50 %.

La composition massique en OPC de l'extrait est de préférence la suivante :

- oligomères :

  ◦ B1 : entre 1 et 4 %, de préférence environ 2,5 %
  o B2 : entre 5 et 9 %, de préférence environ 7,5 %
  o B3 : jusqu'à 3 %, de préférence environ 1,7 %
  o B4 : entre 1 et 4 %, de préférence environ 2,6 %

- Monomères :

  o Catéchine : entre 3 et 6 %, de préférence environ 4,7 %
  o Epicatéchine : entre 12 et 15 %, de préférence environ 13,3 %
  o Epicatéchine 30 gallate : jusqu'à 2 %, de préférence environ 0,7 %

L'extrait aqueux de pépins de raisin selon la présente invention peut être obtenu par extraction aqueuse à l'eau sulfurée de marc de raisin blanc ou rouge, de préférence blanc. Lors de cette extraction, on peut utiliser comme solvant une solution aqueuse d'anhydride sulfureux préparée en dissolvant du métabisulfite de sodium ($Na_2S_2O_3$) dans de l'eau et en ajustant le pH à 3,8 avec de l'acide acétique. On peut ensuite effectuer les étapes suivantes :

- centrifugation pour éliminer les matières en suspension insolubles ;
- filtration pour éliminer notamment l'acide tartrique, les sucres, les polysaccharides et les minéraux ;
- évaporation ;
- puis, éventuellement nouvelle extraction à l'eau sulfurée, suivie d'une centrifugation, filtration, évaporation, jusqu'à récupérer un concentré sous forme liquide ;
- pasteurisation flash ;

- atomisation si l'on souhaite que l'extrait se présente sous forme de poudre.

Selon la présente invention, l'extrait aqueux de pépins de raisin est de préférence associé au fluorure de sodium ou au fluorinol (R1 = H et R2 = $-CH_2OH$ en position 3 par rapport à l'azote). L'extrait aqueux de pépins de raisin est plus préférentiellement associé au fluorinol.

La présente invention a également pour objet l'utilisation d'un extrait aqueux de pépins de raisin en association avec au moins un sel de fluor, notamment ceux décrit précédemment, pour la préparation d'une composition pour hygiène buccale destinée à :

- prévenir la formation du tartre ou l'apparition de maladies bucco-dentaires liées à l'accumulation du biofilm.

En effet, la composition selon la présente invention, outre son effet préventif, peut aussi diminuer l'indice de plaque chez des personnes présentant déjà un biofilm buccal.

En particulier, l'association selon la présente invention permet de prévenir des maladies bucco-dentaires telles que les caries, les gingivites ou les parodontites, dont l'origine est liée à l'accumulation du biofilm.

L'action anti-adhésion de la composition est mise en évidence par la Demanderesse :

- d'une part sur un modèle expérimental de biofilm buccal développé à partir d'une suspension contenant différentes bactéries buccales, sur des disques d'hydroxyapatite ;
- et en montrant, d'autre part, l'activité inhibitrice de ladite composition sur la réaction enzymatique des glucosyltransférases.

La présente invention a également pour objet une composition pour hygiène buccale comprenant une association d'un extrait aqueux de pépins de raisin et d'au moins un sel de fluor, tels que précédemment décrits.

Ainsi, les sels de fluor peuvent être choisis parmi des fluorures inorganiques tels que les fluorures de potassium, de sodium et d'étain, ainsi que leurs mélanges, ou parmi les fluorures d'amine, tels que ceux répondant à la formule (I) suivante :

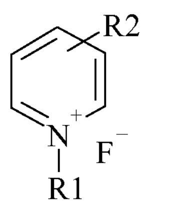

(I)

dans laquelle :

* R1 = H, alcoyle ;

* R2 = $-CH_2-OH$, carboxylate d'alcoyle, $-CO-NH-CH_2-CH_2-OH$,

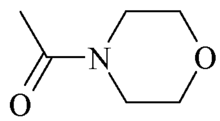

;

et R2 se trouve en position 2 ou 3.

La composition selon la présente invention contient de préférence un extrait aqueux de pépins de raisin en association avec du fluorure de sodium ou du fluorinol. (RI = H et R2 = $-CH_2OH$ en position 3 par rapport à l'azote). L'extrait aqueux de pépins de raisin est plus préférentiellement associé au fluorinol.

La composition selon la présente invention peut se présenter sous la forme d'un bain de bouche, d'un gel ou d'une pâte dentifrice, d'un gel gingival, d'une gomme à mâcher, d'une pastille à sucer, d'un fil dentaire, d'une capsule extrudée, de brins pour brosses à dents, d'un pansement adhésif, d'une lingette imprégnée, d'une pâte adhésive pour dentiers et prothèses ou d'une pâte prophylactique de polissage.

On peut également envisager d'associer à la composition selon la présente invention, au moins un autre actif antibiofilm tel qu'un extrait de canneberge (ou *Vaccinium niacrocarpon*).

Ladite composition peut contenir en outre du siliglycol et/ou de la vitamine E et/ou de la vitamine C.

Ladite composition contient de préférence entre 50 μg/ml et 10000 μg/ml, avantageusement entre 200 μg/ml et 5000 μg/ml et de façon plus avantageuse entre 1000 μg/ml et 3000 μg/ml et de façon encore plus avantageuse environ 2000 μg/ml dudit extrait de pépins de raisin.

Ladite composition contient de préférence entre 50 ppm et 10 000 ppm, plus préférentiellement entre 500 ppm et 2 000 ppm ; et encore plus préférentiellement entre 1 000 ppm et 1 500 ppm de fluor. De façon avantageuse, la composition comprend environ 1 500 ppm de fluor.

De préférence, la composition selon la présente invention contient entre 1000 μg/ml et 3000 μg/ml de l'extrait aqueux de pépins de raisin et entre 500 ppm et 2 000 ppm de fluor, et de manière encore plus préférée environ 2000 μg/ml de l'extrait de pépins de raisin et entre 1 000 ppm et 1 500 ppm de fluor.

La figure 1 représente l'effet de l'extrait de pépins de raisin selon l'exemple 1 en association avec le fluorinol sur la croissance de Streptocoques et *Porphyromonas gingivalis* sur un modèle expérimental de biofilm buccal en action préventive.

La figure 2 représente l'effet de l'extrait de pépins de raisin selon l'exemple 1 en association avec NaF sur la croissance de Streptocoques et *Porphyromonas gingivalis* sur un modèle expérimental de biofilm buccal en action préventive.

La présente invention est illustrée par les exemples suivants.

EXEMPLES

1) Préparation d'un extrait aqueux de pépins de raisins

L'extrait aqueux de pépins de raisin est fourni par la Société Française de Distilleries. Il est obtenu par extraction à l'eau sulfurée de marc de raisin blanc selon les techniques conventionnelles d'extraction, bien connues de l'homme de métier.

Les caractéristiques de l'extrait sont les suivantes :

- Apparence : poudre fine
- Couleur : ocre
- Goût : astringent
- Densité : 0,35 à 0,55 g/ml
- Humidité : < 8 %

L'analyse par HPLC de l'extrait donne la composition suivante :

❖ Oligomères :

- B1 : 2,56 %
- B2 : 7,51 %
- B3 : 1,72%
- B4 : 2,61 %

❖ Monomères:

- Catéchine : 4,69 %
- Epicatéchine : 13,32 %
- Epicatéchine 30 Gallate : 0,66 %

❖ Total OPC identifiés : 33,00 %

2) Action préventive sur un modèle expérimental de biofilm buccal

2.1) Protocole

a) Souches bactériennes et préparation de l'inoculum

Les souches bactériennes utilisées sont :

- *Streptococcus mutants* ATCC 25175
- *Streptococcus sobrinus* ATCC 33478
- *Porphyromonas gingivalis* ATCC 33277

Ces différentes bactéries, en culture dans leur milieu spécifique, sont inoculées dans 10 ml de milieu de culture universel FUM (« Fluid Universal Medium », composition du milieu en annexe 1), puis mises en incubation en anaérobiose à 37°C pendant 24 heures.

Après une vérification microscopique de la pureté, les séries de cultures sont ajustées de façon indépendante à $DO_{550}$ 1,0 +/- 0,05 (soit environ $10^7$ cellules par millilitres) par des dilutions avec du FUM. Des aliquots (1 ml) de chaque culture ajustée à la bonne DO sont réunis constituant ainsi la suspension bactérienne servant à inoculer le milieu à partir duquel va se développer le biofilm.

b) Préparation de la salive

La salive est collectée sans stimulation, chez des volontaires sains (après obtention de leur consentement éclairé), au moins une heure trente après avoir mangé, bu ou s'être brossé les dents.

Les différents échantillons de salive recueillis sont mélangés, centrifugés (30 minutes, 4°C, 15000 tours/minute) et le surnageant est pasteurisé (30 minutes, 65°C) puis à nouveau centrifugé dans des tubes stériles. Ce surnageant ainsi obtenu est réparti en tubes de 50 ml et stocké à -20°C. L'efficacité de la pasteurisation est vérifiée par étalement d'échantillons de salive sur géloses au sang. Après 72 heures de culture à 37°C, aucune CFU n'est observée.

c) Traitement préventif des biofilms

Le biofilm se développe sur des disques d'hydroxyapatite stérilisés par autoclavage à 125°C pendant 20 minutes.

A. La première étape est la formation de la pellicule acquise à la surface des disques. Chaque disque est placé dans un des puits d'une boite de culture stérile 24 puits en polystyrène et mis en incubation avec 800 μl de salive pendant 4 heures, à température ambiante avec une agitation douce.

B. La salive est ensuite aspirée de chaque puits et remplacée par un mélange contenant : 800 μl de salive + 1000 μl de FUM contenant 0.15 % de glucose et 0.15 % de saccharose + 200 μl de la suspension contenant les différentes bactéries buccales (même quantité de chaque).

C. Puis, les différentes quantités des associations à tester sont ajoutées dans les puits afin d'obtenir les concentrations souhaitées (chaque concentration est testée en triplicatat). Voir tableaux 1 et 2.

Tableau 1

| EXPERIENCE n° 1 | | |
|---|---|---|
| Produits | Quantités ajoutées | Concentration finale testée |
| Témoin | 153 μl de sérum physiologique (SP) | |
| Extrait de pépins de raisin selon l'exemple 1 (solution à 100 mg/ml) | 43 μl Extrait de pépins de raisin selon l'exemple 1 + 110 μl SP | 2000 μg/ml |
| Fluorinol (solution à 20%) | 73 μl Fluorinol + 80 μl SP | 0.68% Fluorinol (= 0.1% Fluor) |
| Fluorinol (solution à 20%) | 110 μl Fluorinol + 43 μl SP | 1.02% Fluorinol (= 0.15% Fluor) |
| Extrait de pépins de raisin selon l'exemple 1 (100 mg/ml) + Fluorinol (20%) | 43 μl d'extrait de pépins de raisin selon l'exemple 1 + 73 μl de Fluorinol + 37 μl SP | 2000 μg/ml d'extrait de pépins de raisin selon l'exemple 1 + 0.68% Fluorinol (1000 ppm Fluor) |

(suite)

| EXPERIENCE n° 1 | | |
|---|---|---|
| Produits | Quantités ajoutées | Concentration finale testée |
| Extrait de pépins de raisin selon l'exemple 1 (100 mg/ml) + Fluorinol (20%) | 43 μl d'extrait de pépins de raisin selon l'exemple 1 + 110 μl de Fluorinol | 2000 μg/ml d'extrait de pépins de raisin selon l'exemple 1 + 1.02% Fluorinol (1500 ppm Fluor) |

Tableau 2

| EXPERIENCE n° 2 | | |
|---|---|---|
| Produits | Quantités ajoutées | Concentration finale testée |
| Témoin | 229 μl de sérum physiologique (SP) | |
| Fluorinol (solution à 20%) | 114 μl Fluorinol + 115 μl SP | 1.02% Fluorinol (= 0.15% Fluor) |
| Extrait de pépins de raisin selon l'exemple 1 (100 mg/ml) + Fluorinol (20%) | 45 μl d'extrait de pépins de raisin selon l'exemple 1 + 114 μl de Fluorinol + 45 μl SP | 2000 μg/ml d'extrait de pépins de raisin selon l'exemple 1 + 1.02% Fluorinol (1500 ppm Fluor) |
| NaF (solution à 4%) | 184 μl NaF + 45 μl SP | 0,33 % NaF (1500 ppm Fluor) |
| Extrait de pépins de raisin selon l'exemple 1 (100 mg/ml) + NaF | 45 μl d'extrait de pépins de raisin selon l'exemple 1 + 184 μl NaF | 2000 μg/ml d'extrait de pépins de raisin selon l'exemple 1 + 0,33% NaF (1500 ppm Fluor) |

La boîte 24 puits est ensuite mise en incubation en anaérobiose à 37°C pendant 64h.
D. Analyse des biofilms après 64h d'incubation. Les disques sont lavés avec une solution de sérum physiologique pour éliminer les bactéries non adhérentes. Chaque disque est ensuite placé dans une boîte de Pétri stérile, et la surface du disque est grattée avec une curette stérile (instrument de parodontologie). La surface du disque gratté ainsi que la boîte de pétri sont rincées avec du sérum physiologique. Le volume récupéré de lavage est complété afin d'obtenir un volume final de 1 ml et la suspension cellulaire est vortexée.

Des dilutions sérielles à 1 : 10 de cette suspension cellulaire sont réalisées jusqu'à atteindre des concentrations de $10^{-2}$ et $10^{-4}$, puis 50 μl de ces dilutions sont étalés sur les 4 géloses spécifiques utilisées : mitis salivarius, MRS, wilkins chalgren (WCA) et géloses au sang. Les géloses sont ensuite placées en anaérobiose à 37°C pendant 48-72h puis le nombre de colonies formées pour chaque espèce est compté.

La différenciation des deux espèces bactériennes s'effectue par observation de la morphologie des colonies sur gélose en parallèle avec une observation au microscope. Les géloses mitis salivarius permettent d'obtenir le nombre de streptocoques et les géloses WCA le nombre de *Porphyromonas.*

Le nombre de colonies formées après 48-72 h d'incubation des molécules avec les biofilms bactériens est exprimé, après comptage, en Unités Formant Colonies par Biofilm analysé (ou UFC /Biofilm) par la formule :

$$[\text{Nombre de colonies comptées x facteur de dilution } (10^2 \text{ à } 10^4)] / \text{Volume d'inoculum } (0.05 \text{ ml})$$

Ensuite la moyenne ainsi que l'écart-type des trois résultats de UFC/Biofilm obtenus sont réalisés pour chaque concentration de molécule testée et pour chaque groupe de bactérie analysée. Le graphe (axe des ordonnées logarithmique) est réalisé sur excel en prenant la moyenne des 3 UFC/Biofilm en fonction de la bactérie comptée.

2.2) Résultats / Conclusions

On observe que l'extrait de pépins de raisin selon l'exemple 1 seul entraîne une diminution significative supérieure à 1 $\log_{10}$ du nombre total de bactéries dans le biofilm développé sur les disques d'hydroxyapatite. Voir Figure 1.

L'extrait de pépins de raisin selon l'exemple 1 permet donc de limiter le développement du biofilm.

On observe par ailleurs que l'effet de l'association extrait de pépins de raisin / fluorinol possède une activité

supérieure à 3 $log_{10}$ sur *Streptococcus* et *Porphyromonas* que celui de l'extrait seul. Voir figure 1.

Le fluorinol, non seulement n'interfère pas l'action de l'extrait de pépins de raisin mais potentialise de façon surprenante et inattendue l'effet de l'extrait de pépins de raisin sur *Streptococcus* et *Porphyromonas.*

On observe aussi que l'effet de l'association extrait de pépins de raisin avec NaF diminue le nombre de bactéries de 3 $log_{10}$ au minimum sur *Streptococcus* et *Porphyromonas* que celui de l'extrait seul dans l'expérience n° 1. Voir figure 2.

NaF potentialise donc aussi l'effet de l'extrait de pépins de raisin sur *Streptococcus* et *Porphyromonas.*

Toutefois, sur *Porphyromonas,* l'effet de potentialisation du fluorinol est plus important que celui de NaF.

3) Inhibition des glucosyltransférases (GTF)

3.1) Protocole

Une souche de *Streptococcus sobrinus* ATCC 33478 est placée dans 1 litre de milieu BHI (bouillon coeur-cervelle) enrichi en glucose (10 g/L concentration finale) en anaérobiose à 37°C pendant 18h. Ce milieu bactérien, dont le pH a été ramené à 6.5 à l'aide d'une solution de NaOH 1N, est ensuite centrifugé pendant 30 minutes à 8000 ppm, 4°C. La glucosyltransférase, présente dans le surnageant, est concentrée par précipitation au sulfate d'ammonium. Celui-ci (50% p/v de saturation) est ajouté progressivement au surnageant maintenu à 4°C sous agitation pendant 30 minutes. Le mélange est ensuite centrifugé à 8000 rpm, 4°C pendant 30 minutes. Le culot, riche en glucosyltranférase, est ensuite repris par du tampon phosphate-potassium $K_2HPO_4$ (10 mM, pH 6.5), dialysé (MWC 6-8000 ; largeur 14.6 mm) pendant 16h puis congelé à-20°C. Ceci constitue la solution de GTF purifiée.

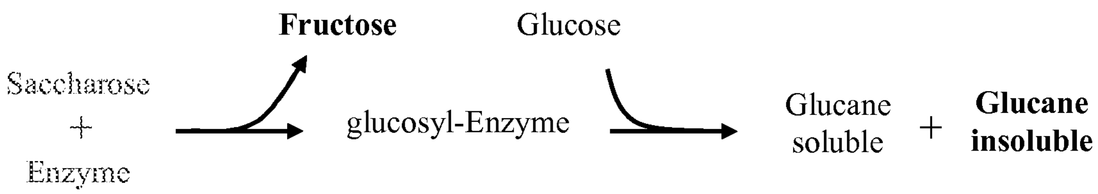


Afin d'évaluer l'action des différents extraits sur l'activité enzymatique des glucosyltransférases, deux mesures sont effectuées : la vitesse initiale de réaction par le dosage du taux de fructose libéré (dosage des sucres réducteurs - test au DNS) et la quantité de glucane insoluble synthétisé (mesures effectuées par des pesées).

Les résultats sont exprimés en pourcentages d'inhibition par rapport à des solutions témoins (eau ultrapure) :

- d'une part le pourcentage d'inhibition de la vitesse initiale de la réaction enzymatique (noté « Fructose » dans le tableau de résultats suivant)
- d'autre part le pourcentage d'inhibition de formation du glucane insoluble produit après 24 heures d'incubation à 37°C (noté « Glucane insoluble » dans le tableau de résultats suivant)

Le mélange réactionnel suivant est placé dans un tube à hémolyse à 37°C:

| | |
|---|---|
| Glucosyltransférase | qsp 0,1 à 0,4 U/ml |
| Saccharose | 50 g/L |
| Tampon phosphate-potassium $K_2HPO_4$ 100 mM | 65 mM, pH 6.5 |
| Azoture de Sodium | 0.1 g/L |
| Dextrane T10 | 2 g/L |
| Polyphenols | 0-2000 μg/ml |

La réaction peut être déclenchée (t=0) par ajout de substrat (saccharose) ou de solution enzymatique.

Des solutions mères de chaque extrait à tester sont préparées par solubilisation des différentes poudres dans de l'eau mQ. A chaque expérience, trois tubes témoins sont réalisés (eau mQ) et chaque concentration d'extrait est testée en triplicatat.

• Dosage des sucres réducteurs : méthode au DNS

Le dextrane interfère sur l'ensemble des méthodes colorimétriques de dosages de protéines, car les particules de dextrane diffractent la lumière et créent une absorbance parasite. La quantité de glucosyltransférase est donc mesurée

par son activité en conditions standards. Une unité glucosyltransférase représente la quantité d'enzyme qui libère une micromole de fructose par minute.

L'activité est déterminée par mesure de la vitesse initiale de production des sucres réducteurs (fructose) à l'aide de la méthode à l'acide dinitro-3,5-salicylique (Sumner et Howell, 1935) avec une gamme étalon en fructose (0-2,5 g/l).

L'acide 3,5 dinitrosalicylique (DNS) de couleur jaune est réduit par les glucides réducteurs en milieu basique à chaud, pour former un complexe de couleur orange-rouge : l'acide 3-amino-5-nitrosalicylique. La méthode présentée permet un dosage de pouvoir réducteur allant jusqu'à l'équivalent de 2,5 g/l de fructose.

Le réactif DNS est préparé de la façon suivante : 150 g de tartrate double de sodium potassium sont dissous dans 250 ml d'eau, puis sont ajoutés 100 ml de NaOH 2N, puis tout en agitant, 1 g d'acide 3,5 dinitrosalicylique. De l'eau mQ est alors additionnée en quantité suffisante pour 500 ml.

Aux temps 0h et 3h, 200 $\mu$L du mélange réactionnel sont prélevés et introduits aussitôt dans des tubes à hémolyse en verre contenant 200 $\mu$L de DNS (arrêt de la réaction). Les sucres réducteurs produits sont alors mesurés par rapport à une gamme étalon fructose (0-2,5 g/l). En parallèle un blanc est réalisé en ajoutant 200 $\mu$L d'eau mQ aux 200 $\mu$L de DNS.

Les tubes sont couverts d'aluminium (DNS sensible à la lumière) puis portés à 90-100°C au bain-marie pendant 5 minutes. L'ensemble est ensuite refroidi rapidement dans la glace pendant 5 minutes. 2 ml d'eau mQ sont alors ajoutés dans tous les tubes et l'absorbance est lue à 540 nm après 20 minutes de repos.

L'activité spécifique est calculée en U/ml de solution d'enzyme, sachant que 1 U est la quantité d'enzyme qui catalyse la formation de 1 $\mu$mol de fructose par minute en conditions standards.

$$\textbf{Activité enzymatique (U/ml)} = \frac{\text{Pente courbe cinétique de dosage}}{\text{Pente courbe étalon fructose}} \; / \; 180 \times 1000 \times \text{facteur de dilutio}$$

• Dosage de la quantité de glucane insoluble formé

La quantité de glucane insoluble synthétisé est mesurée 24h après le début de la réaction enzymatique. Afin de stopper la réaction, les tubes sont couverts d'aluminium puis portés à 90-100°C au bain-marie pendant 5 minutes. L'ensemble est ensuite refroidi rapidement dans la glace pendant 5 minutes. Le milieu (2 ml) est alors centrifugé pendant 30 minutes à 15000 ppm, 4°C.

Le culot est ensuite lavé deux fois à l'eau ultrapure, séché pendant 24h à 65°C puis pesé.

3.2) Résultats et conclusions

Le fluorinol seul aboutit à des pourcentages d'inhibition de la réaction enzymatique initiale de 10.3% et 20.6% pour les concentrations de 0.68% et 1.02% respectivement. De plus, à ces deux concentrations, le fluorinol est inefficace sur la synthèse de glucane insoluble.

L'extrait de pépins de raisin seul à 0.2% entraîne 43.9% d'inhibition de la vitesse initiale de réaction enzymatique et 96.4% d'inhibition de formation de glucane insoluble (GI). L'ajout de 0.68% et 1.02% de fluorinol à cet extrait 0.2% entraîne de meilleurs pourcentages d'inhibition de la vitesse initiale de réaction de 57.4% et 65.7% respectivement.

Les pourcentages d'inhibition de la synthèse de glucane insoluble obtenus avec cette association sont de 96.1% et 97.1% respectivement.

Globalement, le fluorinol permet donc de potentialiser l'activité d'inhibition des GTF de l'extrait de pépins de raisin.

## Revendications

1. Association d'un extrait aqueux de pépins de raisin avec au moins un sel de fluor pour son utilisation en tant que composition anti-adhésion d'hygiène buccale destinée à prévenir et/ou diminuer la formation et l'accumulation du biofilm dentaire sur les surfaces dentaires ou le tissu épithélial gingival, tout en préservant l'équilibre de la flore bactérienne buccale.

2. Association selon la revendication 1, **caractérisée en ce que** ledit sel de fluor est choisi parmi les sels répondant à la formule (I) suivante :

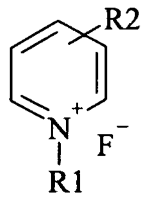

(I)

dans laquelle :

* R1 = H, alcoyle ;
* R2 = $-CH_2-OH$, carboxylate d'alcoyle, $-CO-NH-CH_2-CH_2-OH$,

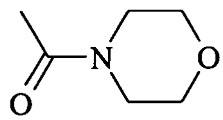

et R2 se trouve en position 2 ou 3.

**3.** Association selon la revendication 2, **caractérisée en ce que** ledit sel de fluor est le fluorhydrate de 3-pyridylméthanol.

**4.** Association selon la revendication 1, **caractérisée en ce que** ledit sel de fluor est le fluorure de sodium.

**5.** Association selon l'une quelconque des revendications 1 à 4, **caractérisée en ce que** ledit extrait aqueux de pépins de raisin contient une fraction massique de polyphénols supérieure à 50 %, de préférence supérieure à 90 %.

**6.** Association selon l'une quelconque des revendications 1 à 5, **caractérisée en ce que** ledit extrait aqueux de pépins de raisin contient une fraction massique de proanthocyanidines comprise entre 20 et 50 %.

**7.** Association selon l'une quelconque des revendications 1 à 6, **caractérisée en ce que** ladite composition pour hygiène buccale est destinée à prévenir la formation du tartre ou l'apparition de maladies bucco-dentaires liées à l'accumulation du biofilm.

**8.** Association selon la revendication 7, **caractérisée en ce que** lesdites maladies bucco-dentaires sont choisies parmi les caries, les gingivites ou les parodontites.

**9.** Composition anti-adhésion pour hygiène buccale comprenant une association d'un extrait aqueux de pépins de raisin et d'un sel de fluor.

**10.** Composition selon la revendication 9, **caractérisée en ce que** le sel de fluor est choisi parmi les sels répondant à la formule suivante :

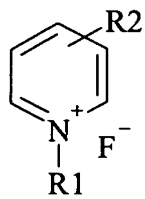

(I)

dans laquelle :

* R1 = H, alcoyle ;
* R2 = $-CH_2-OH$, carboxylate d'alcoyle, $-CO-NH-CH_2-CH_2-OH$,

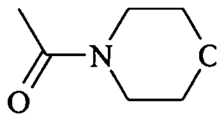

et R2 se trouve en position 2 ou 3.

**11.** Composition selon la revendication 10, **caractérisée en ce que** ledit sel de fluor est le fluorhydrate de 3-pyridylméthanol.

**12.** Composition selon l'une quelconque des revendications 9 à 11, **caractérisée en ce que** qu'elle se présente sous la forme d'un bain de bouche, d'un gel dentifrice, d'un gel gingival, d'une gomme à mâcher, d'une pastille à sucer, d'un fil dentaire, d'une capsule co-extrudée, de brins pour brosse à dents, d'un pansement adhésif, d'une lingette imprégnée, d'une pâte adhésive pour dentiers et prothèses ou d'une pâte prophylactique de polissage.

**13.** Composition selon l'une quelconque des revendications 9 à 12, **caractérisée en ce qu'**elle contient en outre du siliglycol et/ou de la vitamine E.

**14.** Composition selon l'une quelconque des revendications 9 à 13, **caractérisée en ce qu'**elle contient entre 50 μg/ml et 10000 μg/ml, de préférence entre 200 μg/ml et 5000 μg/ml, encore plus avantageusement entre 1000 μg/ml et 3000 μg /ml, encore plus avantageusement environ 2000 μg/ml dudit extrait de pépins de raisin.

**15.** Composition selon l'une quelconque des revendications 9 à 14, **caractérisée en ce qu'**elle contient entre 50 ppm et 10 000 ppm de fluor, de préférence entre 500 ppm et 2 000 ppm de fluor.

**Patentansprüche**

**1.** Verbindung eines wässerigen Extrakts aus Traubenkernen mit wenigstens einem Fluorsalz für seine Verwendung als Anti-Haftzusammensetzung für die Mundhygiene, die zur Vorbeugung und / oder Verringerung der Bildung und Akkumulierung des Zahn-Biofilms auf den Zahnflächen oder dem epithelialen Zahnfleischgewebe bei gleichzeitiger Bewahrung des Gleichgewichts der Bakterienflora des Mundes bestimmt ist.

**2.** Verbindung gemäß Anspruch 1, **dadurch gekennzeichnet, dass** das genannte Fluorsalz aus den Salzen ausgewählt ist, die der folgenden Formel (I) entsprechen:

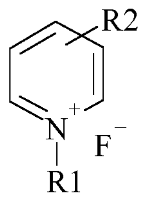

(I)

in der:

*R1 = H, Alkyl;
* R2 = $-CH_2-OH$, Alkyl-Carboxylat, $-CO-NH-CH_2-CH_2-OH$,

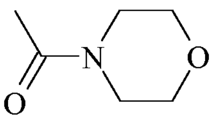

;

und R2 sich in Position 2 oder 3 befindet.

**3.** Verbindung gemäß Anspruch 2, **dadurch gekennzeichnet, dass** das genannte Fluorsalz 3-Pyridylmethanol-Fluorhydrat ist.

**4.** Verbindung gemäß Anspruch 1, **dadurch gekennzeichnet, dass** das genannte Fluorsalz Natriumfluorid ist.

**5.** Verbindung gemäß Anspruch 1 bis 4, **dadurch gekennzeichnet, dass** der genannte wässerige Extrakt aus Traubenkernen einen Masseprozentsatz an Polyphenolen von mehr als 50 %, bevorzugt mehr als 90 % enthält.

**6.** Verbindung gemäß Anspruch 1 bis 5, **dadurch gekennzeichnet, dass** der genannte wässerige Extrakt aus Traubenkernen einen Masseprozentsatz an Proanthocyanidinen enthält, der zwischen 20 und 50 % inbegriffen ist.

**7.** Verbindung gemäß Anspruch 1 bis 6, **dadurch gekennzeichnet, dass** die genannte Zusammensetzung für die Mundhygiene dazu bestimmt ist, die Bildung von Zahnstein oder das Auftreten von Mund- und Zahnkrankheiten im Zusammenhang mit der Akkumulierung des Biofilms zu verhindern.

**8.** Verbindung gemäß Anspruch 7, **dadurch gekennzeichnet, dass** die genannten Mund- und Zahnkrankheiten aus Karies, Zahnfleischentzündungen oder Parodontitis ausgewählt sind.

**9.** Anti-Haftzusammensetzung für die Mundhygiene, umfassend eine Verbindung eines wässerigen Extrakts aus Traubenkernen und einem Fluorsalz.

**10.** Zusammensetzung gemäß Anspruch 9, **dadurch gekennzeichnet, dass** das Fluorsalz ausgewählt ist aus den Salzen, die der folgenden Formel (I) entsprechen:

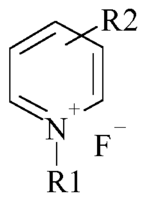

(I)

in der:

* R1 = H, Alkyl;
* R2 = $-CH_2-OH$, Alkyl-Carboxylat, $-CO-NH-CH_2-CH_2-OH$,

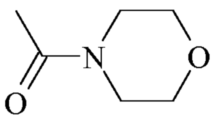

;

und R2 sich in Position 2 oder 3 befindet.

**11.** Zusammensetzung gemäß Anspruch 10, **dadurch gekennzeichnet, dass** das genannte Fluorsalz 3-Pyridylmethanol-Fluorhydrat ist.

**12.** Zusammensetzung gemäß Anspruch 9 bis 11, **dadurch gekennzeichnet, dass** es die Form einer Mundspülung, eines Zahnpastagels, eines Zahnfleischgels, eines Kaugummis, einer Lutschpastille, einer Zahnseide, einer koextrudierten Kapsel, von Borsten für Zahnbürsten, eines Haftpflasters, eines imprägnierten Feuchttuchs, einer Haftpaste für Gebisse und Prothesen oder einer prophylaktischen Polierpaste aufweist.

**13.** Zusammensetzung gemäß Anspruch 9 bis 12, **dadurch gekennzeichnet, dass** sie darüber hinaus sililycol und / oder Vitamine E enthält.

**14.** Zusammensetzung gemäß Anspruch 9 bis 13, **dadurch gekennzeichnet, dass** sie zwischen 50 µg/ml und 10.000

μg/ml, bevorzugt zwischen 200 μg/ml und 5.000 μg/ml, vorteilhafter zwischen 1.000 μg/ml und 3.000 μg/ml, noch vorteilhafter ungefähr 2.000 μg/ml des genannten Extrakts aus Traubenkernen enthält.

**15.** Zusammensetzung gemäß Anspruch 9 bis 14, **dadurch gekennzeichnet, dass** sie zwischen 50 ppm und 10.000 ppm Fluor, bevorzugt zwischen 500 ppm und 2.000 ppm Fluor enthält.

## Claims

**1.** Combination of an aqueous grape seed extract with at least one fluorine salt for use as a buccal hygiene anti-adhesion composition intended to prevent and/or reduce the formation and accumulation of dental biofilm on dental surfaces or on gingival epithelial tissue, whilst maintaining the balance of buccal bacterial flora.

**2.** Combination according to claim 1, **characterized in that** said fluorine salt is selected from salts fitting the following formula (I);

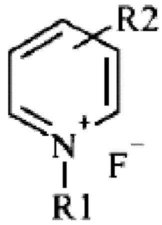

(I)

wherein:

* R1 = H, alkyl;
* R2 = $CH_2$-OH, alkyl carboxylate, -CO-NH-$CH_2$-$CH_2$-OH,

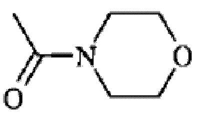

;

and R2 is found in position 2 or 3.

**3.** Combination according to claim 2, **characterized in that** said fluorine salt is 3-pyridyl methanol fluorohydrate.

**4.** Combination according to claim 1, **characterized in that** said fluorine salt is sodium fluoride.

**5.** Combination according to any of claims 1 to 4, **characterized in that** said aqueous grape seed extract contains a mass fraction of polyphenols greater than 50%, preferably greater than 90%.

**6.** Combination according to any of claims 1 to 5, **characterized in that** said aqueous grape seed extract contains a mass fraction of proanthocyanidins of between 20 and 50%.

**7.** Combination according to any of claims 1 to 6, **characterized in that** said composition for buccal hygiene is intended to prevent formation of tartar or the occurrence of bucco-dental diseases related to accumulation of biofilm.

**8.** Combination according to claim 7, **characterized in that** said bucco-dental diseases are selected from caries, gingivitises or parodontitises.

**9.** Anti-adhesion composition for buccal hygiene comprising a combination of an aqueous grape seed extract and of a fluorine salt.

**10.** Composition according to claim 9, **characterized in that** the fluorine salt is selected from salts fitting the following

formula (I);

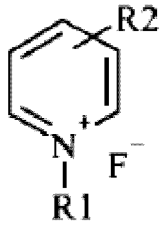

(I)

wherein:

* R1 = H, alkyl;
* R2 = $CH_2$-OH, alkyl carboxylate, -CO-NH-$CH_2$-$CH_2$-OH,

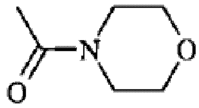

;

and R2 is found in position 2 or 3.

**11.** Composition according to claim 10, **characterized in that** said fluorine salt is 3-pyridylmethanol fluorohydrate.

**12.** Composition according to any of claims 9 to 11, **characterized in that** it is presented as a mouthwash, a toothpaste, a gingival gel, a chewing gum, a suckable tablet, dental floss, an extruded capsule, toothbrush bristles, an adhesive bandage, an impregnated towelette, an adhesive paste for dentures and prostheses or a prophylactic polishing paste.

**13.** Composition according to any of claims 9 to 12, **characterized in that** it further contains siliglycol and/or vitamin E.

**14.** Composition according to any of claims 9 to 13, **characterized in that** it contains between 50 μg/mL and 10,000 μg/mL, preferably between 200 μg/mL and 5,000 μg/mL, still more advantageously between 1,000 μg/mL and 3,000 μg/mL, and still more advantageously about 2,000 μg/mL of said grape seed extract.

**15.** Composition according to any of claims 9 to 14, **characterized in that** it contains between 50 ppm and 10,000 ppm of fluorine, preferably between 500 ppm and 2,000 ppm of fluorine.

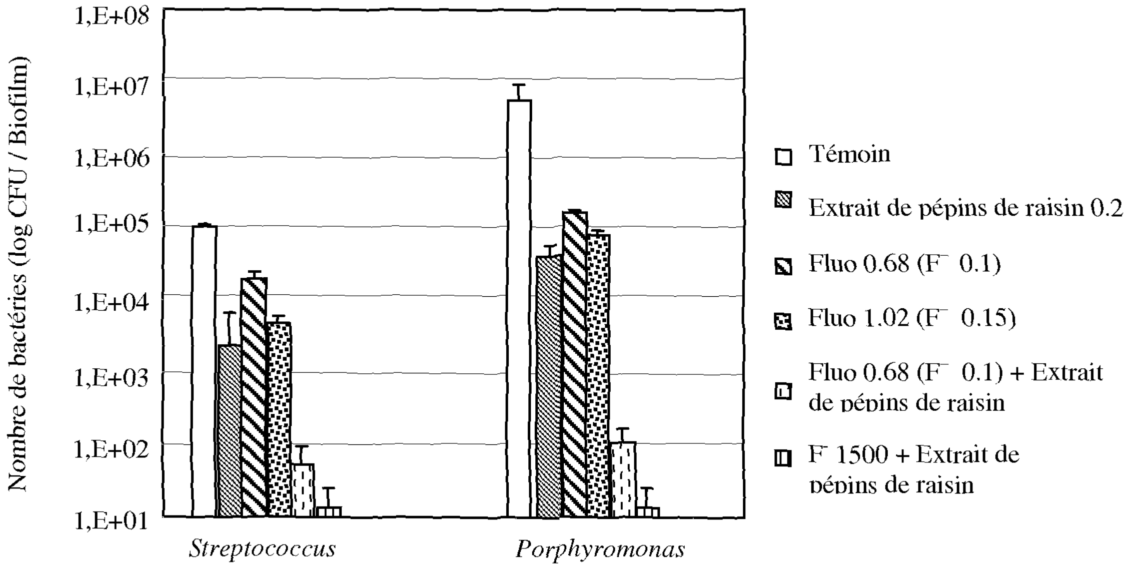


FIGURE 1

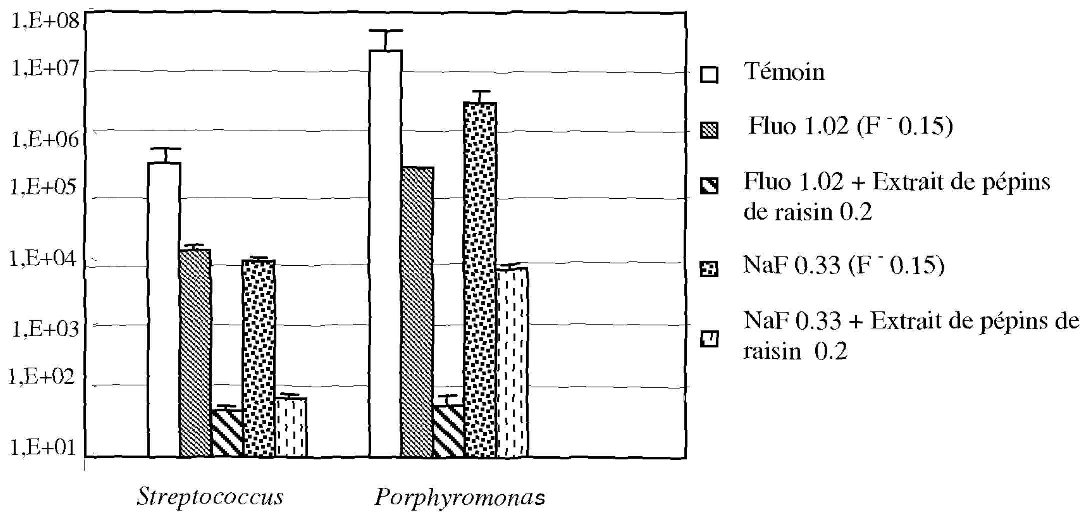


FIGURE 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1072254 A
- US 5470565 A
- FR 2318632
- DE 19949575
- US 20030103914 A